# EUROPEAN PATENT SPECIFICATION

(11) **EP 0 549 725 B1**
(45) Date of publication and mention of the grant of the patent: **08.01.1997**
(21) Application number: 91919186.6
(22) Date of filing: 09.09.1991
(51) Int. Cl.: A61M 25/01

(54) **ADJUSTABLE CATHETER CONTAMINATION SHIELD**
EINSTELLBARE KONTAMINATIONSHÜLSE FÜR KATHETER
PROTECTION REGLABLE CONTRE LA CONTAMINATION POUR CATHETER

(30) Priority: 21.09.1990 US 586626
(43) Date of publication of application: 07.07.1993
(60) Divisional application: 96200352.1
(73) Proprietor: BAXTER INTERNATIONAL INC., Deerfield, IL 60015 (US)
(72) Inventor: WOODGRIFT, Randal, W., Laguna Niguel, CA 92677 (US); WELSH, Gregory, P., Newport Beach, CA 92663 (US)
(74) Representative: MacGregor, Gordon
(86) International application number: US9106517
(87) International publication number: WO9204932

(56) References cited:
- EP-A- 0 311 427
- FR-A- 2 120 413
- US-A- 4 327 723
- US-A- 4 767 409

## Description

The present invention generally relates to apparatus for preventing the contamination of a catheter as it is inserted into and withdrawn from a body cavity or lumen. More particularly, the present invention is directed to apparatus including an expandable shield for providing a sterile environment for a catheter enclosed thereby. In view of commonly accepted procedures for the utilization of catheters within a venous lumen, such as balloon-tipped catheters, which include the adjustment of catheter depth, or inserted length, it is most desirable to retain sterility of the exposed catheter portion exterior to the body.

A number of prior art devices have addressed this problem and utilized a protective sheath such as a thin, plastic, flexible and collapsible hollow sleeve, which is disposed exterior to the catheter and supported between distal and proximal fittings. For example, see US-A-4327723 and US-A-4515592. The sleeve disclosed in US-A-4515592 is caused to fold concertina fashion when the fittings are relatively moved towards each other.

In these patents the catheter may be inserted through the proximal and distal fittings and a collapsed sleeve before insertion into a venous lumen or the like. The collapsed sleeve may have a length of up to ten centimeters, in view of the fact that it is desirable for it to be expanded to lengths as long as one meter for providing a sterile environment for the catheter. To facilitate the insertion of the catheter through the collapsed sleeve, prior devices as in these patents have provided a length of tube approximately equal to the length of collapsed sleeve and disposed between proximal and distal fittings to provide a passage through the collapsed sleeve and prevent rupture, or puncture of the collapsed sleeve during insertion of the catheter therethrough. Once the distal and proximal fittings of the prior art device are pulled apart, and disconnected from the interconnecting tube, the sleeve is expanded and reinsertion of the catheter through the sleeve is difficult, if not impossible, due to difficulty in mutual alignment of the proximal and distal ends.

This is due to the fact that the prior art sleeves do not expand in a controlled manner in which the sleeve is divided into a totally collapsed portion and a totally expanded portion, the latter enabling visual observation of the catheter therethrough. Rather, in prior art devices the expansion of the sleeve is uncontrolled which results in all of the sleeve being partially expanded between the distal and proximal fittings thus obscuring visual observation of the catheter therethrough.

The present invention provides an adjustable catheter contamination shield as in Claim 1, which enables the alignment of the proximal and distal ends without separate interconnection thereof by a tube or the like.

The precharacterising part of Claim 1 is based on EP-A-0311427, which discloses an adjustable catheter contamination shield comprising a distal fitting including bushing means for coupling to an introducer and means, defining a bore through the distal fitting, for enabling passage of a catheter therethrough; a proximal fitting including means, defining a bore therethrough, for enabling passage of the catheter through the proximal fitting and collapsible shield means, disposed between said distal and proximal fittings, for enclosing an adjustable space therebetween and for shielding a portion of the catheter disposed between the distal and proximal fittings.

The present invention is distinguished by supporting means for supporting the shield means in a collapsed configuration and for causing the collapsed shield means to expand from only one portion of the collapsed configuration when the distal and proximal fittings are moved apart from one another.

The collapsible shield means prevents contact with a portion of the catheter disposed between the distal and the proximal fittings.

The collapsible shield means may be formed from a material enabling visual observation of the catheter through an expanded portion thereof when the expanded portion is in a state of tension.

To prevent stress on the catheter passing through the proximal and distal fittings, each fitting may be provided with a flexible tube connected thereto for preventing forces being applied to the catheter during manipulation of the proximal and distal fittings during insertion of the catheter therethrough and during manipulation of the catheter as it is inserted and withdrawn from a venous lumen.

### BRIEF DESCRIPTION OF THE DRAWINGS

A better understanding of the present invention may be had by consideration of the following detailed description, taken in conjunction with the accompanying drawings, in which:
Figure 1 is a side view of an adjustable catheter contamination shield, in accordance with the present invention, depicting its use with an introducer for introducing a catheter into a heart;
Figure 2 is an exploded pictorial representation of the contamination shield shown in Figure 1;
Figure 3 is a side view of the shield of Fig.1, shown in fully collapsed condition;
Figure 4 is a side view of the shield shown in Figure 3 with the catheter inserted therethrough;
Figure 5 is a side view of the shield shown in Figure 3 with a portion of the shield shown in an extended state with the catheter visible therethrough;
Figure 6 is a side view of the shield shown in Figure 3 in a totally expanded condition.
Figure 7 is a cross-sectional view of a portion of a proximal fitting.
Figure 8 is a side view of an alternative proximal fitting;
Figure 9 shows a portion of the proximal fitting bent through approximately 90°;
Figure 10 is a view of the fitting of Fig.8;

### DETAILED DESCRIPTION OF THE DRAWINGS

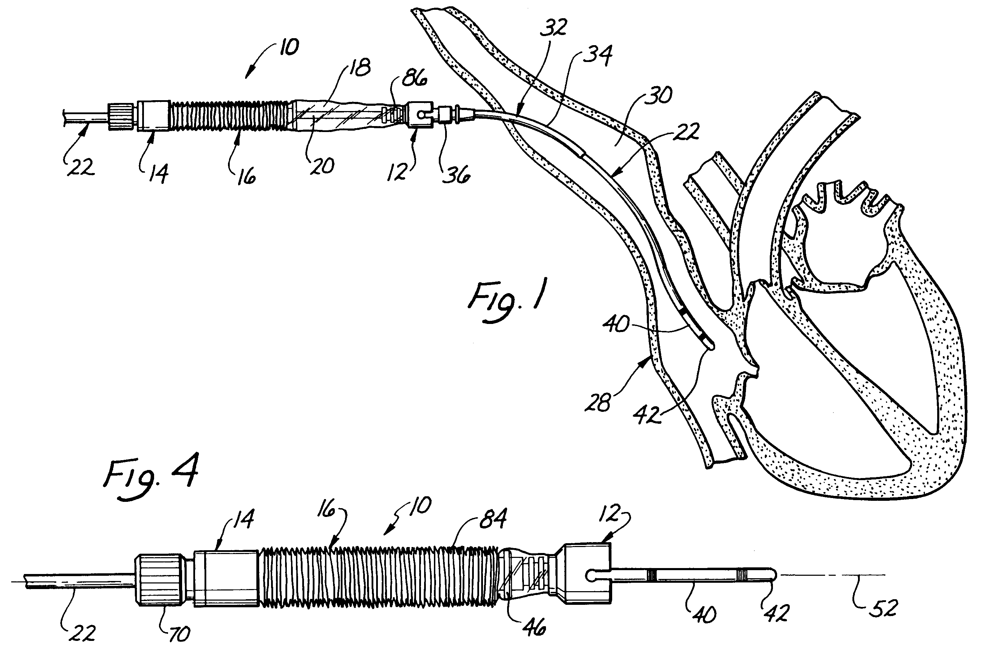

Turning now to Figures 1 to 6, there is shown an adjustable catheter contamination shield 10 generally including a distal fitting 12, a proximal fitting 14, and a collapsible shield 16, disposed between the distal and proximal fittings 14, 12 which provides means for enclosing an adjustable space 18 therebetween and for preventing contact with a portion 20 of a catheter 22 disposed between the distal and proximal fittings 12, 14. As diagrammatically shown in Figure 1, the catheter contamination shield is positioned for the insertion of the catheter 22 into a heart 28 through an internal jugular vein 30 by means of an introducer 32 which includes an introducer sheath 34 and a fitting 36, the introducer 32, including the sheath 34 and fitting 36 being of conventional design. As shown, the catheter 22 may include a balloon portion 40 disposed proximate a tip 42, the balloon portion being commonly used and well-known in the art.

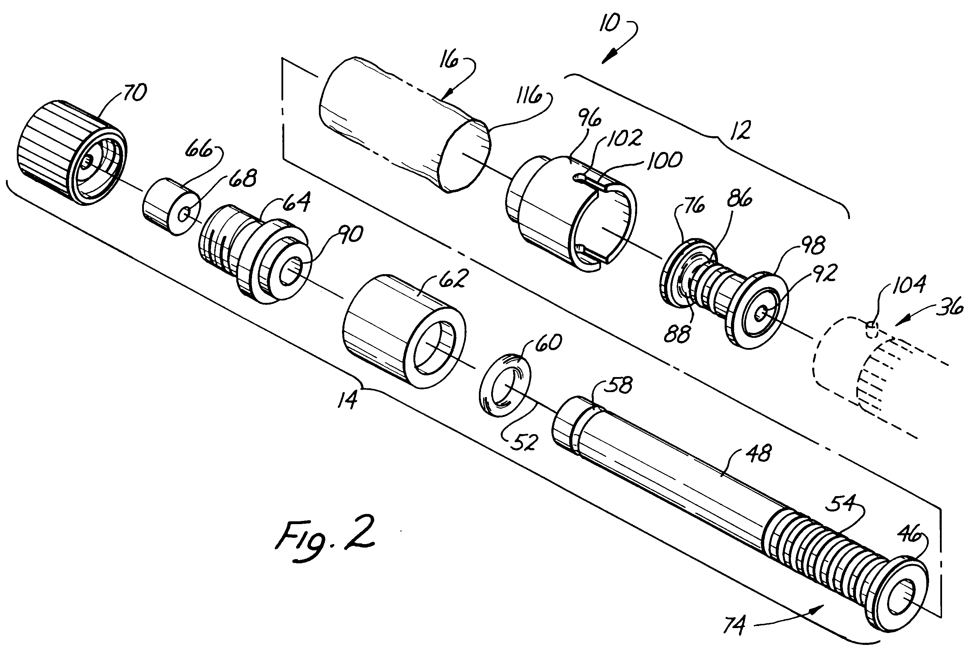

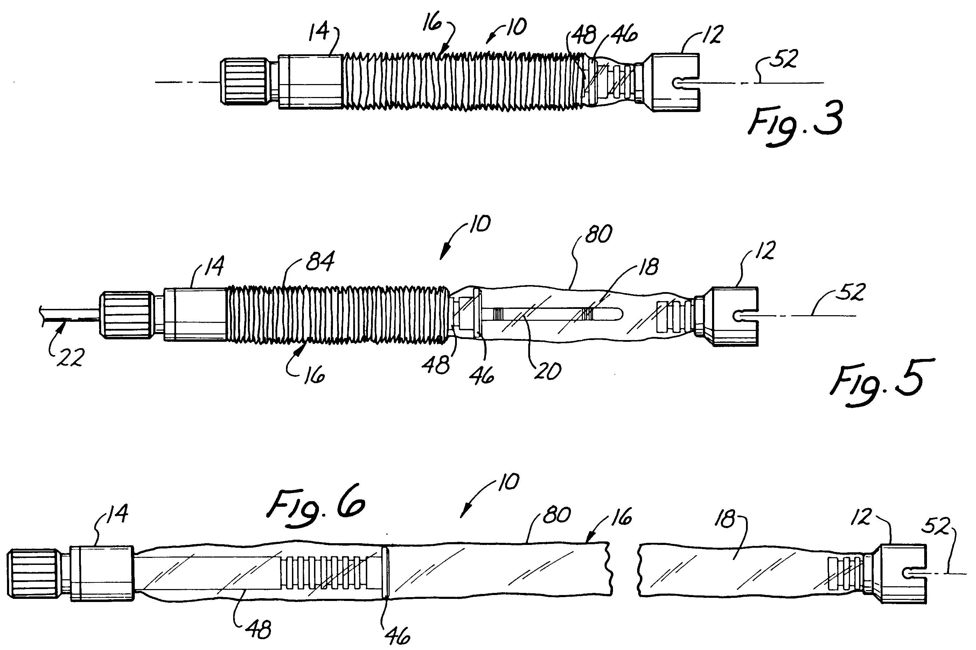

Figure 2 is an exploded perspective view of the catheter contamination shield 10 showing in greater detail the distal fitting 12 and the proximal fitting 14. A flange 46 formed as an integral portion of a tube 48 provides a means for supporting the shield 16 in the collapsed configuration and, in addition, causing the collapsed shield to expand along a longitudinal axis 52 as shown in Figures 3, 4, 5 and 6 and hereinafter described in greater detail.

It should be appreciated that, while the flange 46 and tube 48 are shown proximate and connected to the proximal fitting 14, alternatively the flange 46 with tube 48 may be connected with the distal fitting 12, such alternative arrangement not being shown in the drawings. The tube 48, which may be formed from any suitable flexible material and may include a ribbed portion 54 proximate the flange 46, not only provides a passage through the collapsed shield 16 bit also importantly provides a means for reducing stress on the catheter passing therethrough which may occur through manual manipulation of the catheter contamination shield 10 and catheter 22 as the latter is advanced through a venous lumen such as the jugular vein 30 as shown in Figure 1.

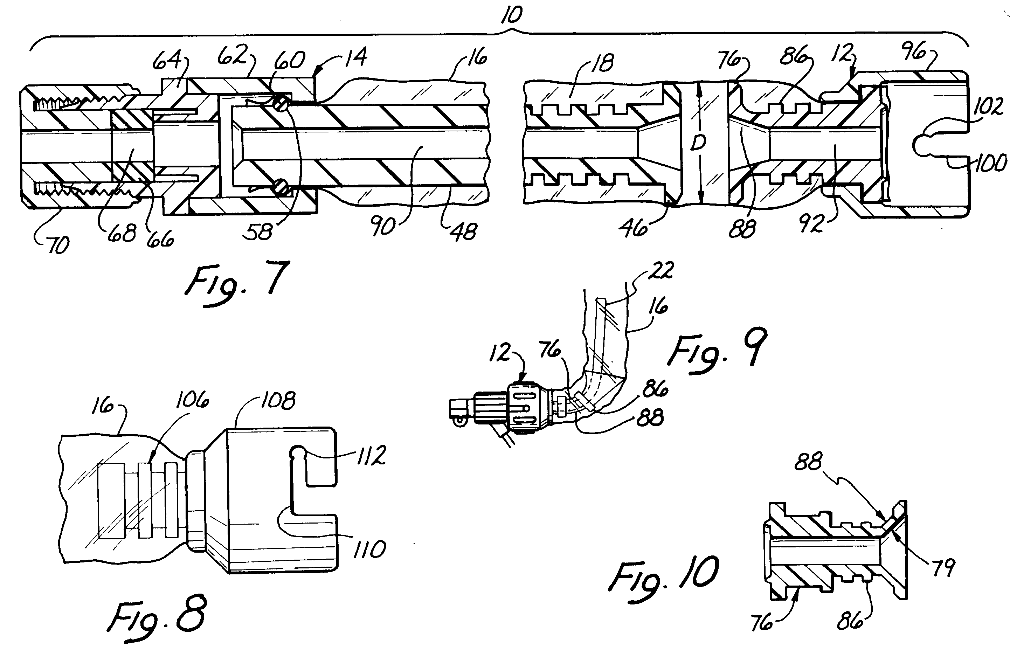

A groove 58 disposed in the flexible tube 48 along with an O-ring 60 and a sleeve 62 provides means for fixing the collapsible shield 16 to the proximal fitting 14 as more clearly shown in cross-section in Figure 7.

Coupled to the sleeve 62 is a threaded fitting 64 enclosing an end bushing 66, having an orifice 68 therethrough sized for fitting into the catheter 22 and held in position by a threaded cap 70 adapted for threaded engagement with the fitting 64 with the bushing 66 there-enclosed, as shown in Figure 7.

As best shown in Figures 5, 6 and 7, the flange 46 has an outside diameter D which is sufficient to prevent the passage of the shield 16 therepast without movement of the distal and proximal fittings 12, 14 from one another. Depending upon the material of construction of the shield 16, this function of the flange 46 in both enabling selected expansion of the shield therepast and for supporting the shield in a collapsed configuration may be obtained when the flange diameter D is greater than the inside diameter of the shield 16. It should be appreciated that while the flange is shown disposed at an end 74, it may be disposed at any intermediate position or a number of flanges, not shown, may be disposed along the tube 48 for controlling the expansion of the shield 16 therepast. The tube 48 provides a clear passage through the collapsed shield 16 and additionally when the shield 16 is collapsed over the tube 48, the flange 46 is coaxially aligned with a flexible distal tube 76 thereby facilitating passage of the catheter 22 therethrough.

The shield 16 may be formed of a polyethylene type material or any other material facilitating the accordion-like collapse thereof over the tube 48 and also, importantly, enabling the visual observation of the catheter 22 through an expanded portion 80 thereof as shown in Figures 5 and 6.

The flange 46 hereinabove described in conjunction with providing a means for maintaining the shield in a selected, collapsed configuration also performs the function of providing tension in the expanded portion 80 between the flange 46 and the distal fitting 12 to enable visual observation of the catheter 22 disposed therein. This state of tension is sufficient to remove a pleating 84 of the shield 16 sufficient to enable the hereinabove visual observation of the catheter. This feature is important in that once the distal and proximal fittings 12, 14 are moved to a spaced apart relationship, the catheter 22 can still be "threaded" or passed from the proximal fitting 14 through the distal fitting 12 by visual alignment thereof through the taut portion 80 of the shield 16. Hence, the structure of the present invention enables the function of threading the catheter 22 through spaced apart proximal and distal fittings 12, 14 which results in a shield providing far greater capability with regard to movement and withdrawal of a catheter than was heretofore possible.

As shown in Figures 2 and 9, the distal fitting 12 includes a distal tube 76, formed of a flexible material similar to that utilized in the tube 48 and also may include a ribbed portion 86 which provides a means for preventing stress on the catheter 22 during manipulation thereof as hereinabove discussed in connection with the proximal tube 48 and the ribbed portion thereof 54. The threaded fitting 64 and the distal tube 76 include bores 90, 92 for enabling passage of the catheter 22 therethrough. In addition, the distal fitting 12 includes a bushing 96 sized for passing over the tube 76 and sealably engaging a flange 98 disposed on the tube 76 to the introducer 32 by way of the introducer fitting 36. The bushing 96 may include a slot 100 and detent 102 for engaging a prong 104 on the introducer fitting 36 in a conventional manner. Alternatively, as shown in Figure 8, an alternative bushing 108 may include a bayonet-type slot 110 with detent 112 for engaging the prong 104 in a conventional manner.

As shown in Figure 9, the distal fitting 12 can be bent up to approximately 90° without kinking of the catheter therein.

A distal end 116 of the shield 16 may be fixed to the distal fitting 12, for a compressed fit between the flange 98 and the bushing 96.

A procedure in accordance with the present invention, and utilizing the structure thereof, provides a sterile environment exterior to a body, such as a heart 28, for the catheter 22 which includes steps of coupling the distal fitting 12 of the contamination shield 10 to an introducer 32, the introducer being adapted for insertion into a venous lumen such as the jugular vein 30 as hereinbefore described.

Thereafter, the procedure in accordance with the present invention includes passing catheter 22 through the proximal fitting 14 and into the sterile area 18 enclosed by the expandable shield 16 disposed between the distal and proximal fittings 12 and 14.

Thereafter, the shield 16 is expanded from the flange 46 to enable visual observation of the catheter portion 20 passing between the distal and proximal fittings 12, 14.

## Claims

1. An adjustable catheter contamination shield comprising
a distal fitting (12) including bushing means (96) for coupling to an introducer (32) and means (76), defining a bore (92) through the distal fitting, for enabling passage of a catheter (22) therethrough;
a proximal fitting (14) including means (48), defining a bore (90) therethrough, for enabling passage of the catheter through the proximal fitting; and
collapsible shield means (16), disposed between said distal and proximal fittings, for enclosing an adjustable space (18) therebetween and for shielding a portion of the catheter disposed between the distal and proximal fittings;
characterised by supporting means (46) for supporting the shield means (16) in a collapsed configuration and for causing the collapsed shield means to expand from only one portion of the collapsed configuration when the distal and proximal fittings are moved apart from one another.

2. An adjustable catheter contamination shield according to Claim 1 wherein said collapsible shield means (16) is formed of a material enabling visual observation of the catheter (22) through said expanded portion of the shield means, when the catheter is disposed between the distal and proximal fittings (12, 14); and said supporting means (46) is operable to produce the expanded portion, while maintaining a remainder of the shield means in the collapsed configuration, as the distal and proximal fittings are moved apart from one another.

3. An adjustable catheter contamination shield according to Claim 2, wherein the shield means (16) enables visual observation of the catheter (22) through the expanded portion thereof when the expanded portion is in a state of tension; and said supporting means (46) is connected with one of said distal and proximal fittings (12, 14), for supporting the shield means in a collapsed configuration with said distal and proximal fittings adjacent to one another for enabling the catheter to be threaded through the bores of the distal and proximal fittings, for causing the collapsed shield means to expand along said longitudinal axis when the distal and proximal fittings are moved apart from one another, and for causing the expanded portion of the shield to be in the state of tension enabling visual observation of the catheter therethrough.

4. An adjustable catheter contamination shield as in any preceding claim wherein the means (76) defining a bore (92) through the distal fitting is a first flexible tube means (76), coaxially aligned with said distal fitting (12), for preventing stress to said catheter (22); and the means (48) defining the bore (90) through the proximal fitting (14) is a second flexible tube means (48), coaxially aligned with said proximal fitting, for preventing stress to said catheter; said collapsible shield means (16) being disposed over said first and second flexible tube means (76, 48).

5. The adjustable catheter contamination shield according to Claim 4 wherein the second flexible tube means (48) includes said supporting means (46), in use, the supporting means (46) causing the collapsed shield means (16) to expand along a longitudinal axis thereof from one end of the collapsed configuration when the distal and proximal fittings (12, 14) are moved apart from one another.

6. The adjustable catheter contamination shield according to any preceding claim wherein said supporting means (46) is disposed proximate said proximal fitting (14).

7. The adjustable catheter contamination shield according to any preceding Claim wherein said supporting means (46) is interconnected with said proximal fitting (14).

8. The adjustable catheter contamination shield according to any preceding claim wherein said supporting means (46) is disposed within said shield means (16).

9. The adjustable catheter contamination shield according to any preceding claim wherein said supporting means comprises a flange (46) having an outside diameter (D) sufficient to prevent passage of the shield means (16) thereover without movement of the distal and proximal fittings (12, 14) from one another.

10. The adjustable catheter contamination shield according to Claim 9 wherein the supporting means further comprises a, or said second, flexible tube means (48), disposed between said flange (46) and proximal fitting (14) and coaxially aligned therewith, for providing a clear passage through the collapsed shield means (16) and for reducing stress on a catheter (22) passing therethrough.

11. The adjustable catheter contamination shield according to any preceding claim wherein said distal fitting (12) includes flexible tube means (76) coaxially aligned therewith for reducing stress on a catheter (22) passing therethrough.

12. The adjustable catheter contamination shield according to any one of Claims 1 to 4, or any one of Claims 6 to 11 when dependent from any one of Claims 1 to 4, Claim, wherein said one portion is positioned between the distal and proximal fittings (12, 14).

13. The adjustable catheter contamination shield according to any one of Claims 1 to 11, wherein said one portion is disposed at an end of the collapsed configuration.

14. A procedure for providing a sterile environment exterior to a body for a catheter (22) comprising the steps of:
coupling a distal fitting (12) for a contamination shield (16) to an introducer (32), the introducer being adapted for insertion into a venous lumen (30);
passing the catheter through a proximal fitting (14) and into a sterile area (18) enclosed by an expandable shield (16) disposed between said distal and proximal fittings;
expanding the shield from one end of a collapsed configuration thereof to enable visual observation of a catheter portion passing between the distal and proximal fittings.

## Patentansprüche

1. Einstellbare Katheterkontaminationsabschirmung, die folgendes aufweist:
ein distales Anschlußstück (12), das eine Hülseneinrichtung (96) zum Koppeln mit einer Einführeinrichtung (32) und eine Einrichtung (76) aufweist, die eine Bohrung (92) durch das distale Anschlußstück definiert, um den Durchtritt eines Katheters (22) durch es hindurch zu ermöglichen;
ein proximales Anschlußstück (14), das eine Einrichtung (48) aufweist, die eine Bohrung (90) durch sie hindurch definiert, um den Durchtritt des Katheters durch das proximale Anschlußstück zu ermöglichen; und
eine kollabierbare Abschirmeinrichtung (16), die zwischen dem distalen und dem proximalen Anschlußstück angeordnet ist, um einen einstellbaren Raum (18) dazwischen zu umschließen und um einen Bereich des Katheters, der zwischen dem distalen und dem proximalen Anschlußstück angeordnet ist, abzuschirmen;
gekennzeichnet durch eine Abstützeinrichtung (46), um die Abschirmeinrichtung (16) in einer kollabierten Konfiguration abzustützen und um zu bewirken, daß sich die kollabierte Abschirmeinrichtung von nur einem Bereich der kollabierten Konfiguration ausgehend ausdehnt, wenn das distale und das proximale Anschlußstück voneinander weg bewegt werden.

2. Einstellbare Katheterkontaminationsabschirmung nach Anspruch 1,
wobei die kollabierbare Abschirmeinrichtung (16) aus einem Material gebildet ist, das eine Sichtbeobachtung des Katheters (22) durch den ausgedehnten Bereich der Abschirmeinrichtung ermöglicht, wenn der Katheter zwischen dem distalen und dem proximalen Anschlußstück (12, 14) angeordnet ist; und die Abstützeinrichtung (46) betätigbar ist, um den ausgedehnten Bereich zu erzeugen, während sie gleichzeitig einen Rest der Abschirmeinrichtung in der kollabierten Konfiguration hält, während das distale und das proximale Anschlußstück voneinander weg bewegt werden.

3. Einstellbare Katheterkontaminationsabschirmung nach Anspruch 2,
wobei die Abschirmeinrichtung (16) eine Sichtbeobachtung des Katheters (22) durch den ausgedehnten Bereich davon ermöglicht, wenn der ausgedehnte Bereich in einem gespannten Zustand ist; und die Abstützeinrichtung (46) mit einem von dem distalen und dem proximalen Anschlußstück (12, 14) verbunden ist, um die Abschirmeinrichtung in einer kollabierten Konfiguration zu halten, wobei das distale und das proximale Anschlußstück einander benachbart sind, um zuzulassen, daß der Katheter durch die Bohrungen des distalen und des proximalen Anschlußstücks geführt wird, um zu bewirken, daß sich die kollabierte Abschirmeinrichtung entlang der Längsachse ausdehnt, wenn das distale und das proximale Anschlußstück voneinander weg bewegt werden, und um zu bewirken, daß der ausgedehnte Bereich der Abschirmung in dem gespannten Zustand ist, der eine Sichtbeobachtung des Katheters durch ihn hindurch ermöglicht.

4. Einstellbare Katheterkontaminationsabschirmung nach einem der vorhergehenden Ansprüche,
wobei die Einrichtung (76), die eine Bohrung (92) durch das distale Anschlußstück definiert, eine erste flexible Rohreinrichtung (76) ist, die koaxial mit dem distalen Anschlußstück (12) ausgefluchtet ist, um eine mechanische Beanspruchung des Katheters (22) zu verhindern;
und die Einrichtung (48), die die Bohrung (90) durch das proximale Anschlußstück (14) definiert, eine zweite flexible Rohreinrichtung (48) ist, die koaxial mit dem proximalen Anschlußstück ausgefluchtet ist, um eine mechanische Beanspruchung des Katheters zu verhindern,
wobei die kollabierbare Abschirmeinrichtung (16) über der ersten und der zweiten flexiblen Rohreinrichtung (76, 48) angeordnet ist.

5. Einstellbare Katheterkontaminationsabschirmung nach Anspruch 4,
wobei die zweite flexible Rohreinrichtung (48) die Abstützeinrichtung (46) aufweist, wobei im Gebrauch die Abstützeinrichtung (46) bewirkt, daß sich die kollabierte Abschirmeinrichtung (16) entlang einer Längsachse davon von einem Ende der kollabierten Konfiguration ausgehend ausdehnt, wenn das distale und das proximale Anschlußstück (12, 14) voneinander weg bewegt werden.

6. Einstellbare Katheterkontaminationsabschirmung nach einem der vorhergehenden Ansprüche,
wobei die Abstützeinrichtung (46) nahe dem proximalen Anschlußstück (14) angeordnet ist.

7. Einstellbare Katheterkontaminationsabschirmung nach einem der vorhergehenden Ansprüche,
wobei die Abstützeinrichtung (46) mit dem proximalen Anschlußstück (14) verbunden ist.

8. Einstellbare Katheterkontaminationsabschirmung nach einem der vorhergehenden Ansprüche,
wobei die Abstützeinrichtung (46) in der Abschirmeinrichtung (16) angeordnet ist.

9. Einstellbare Katheterkontaminationsabschirmung nach einem der vorhergehenden Ansprüche,
wobei die Abstützeinrichtung einen Flansch (46) aufweist, der einen Außendurchmesser (D) hat, der ausreicht, um das Darüber-Hinwegbewegen der Abschirmeinrichtung (16) ohne eine Bewegung des distalen und des proximalen Anschlußstücks (12, 14) voneinander weg zu verhindern.

10. Einstellbare Katheterkontaminationsabschirmung nach Anspruch 9,
wobei die Abstützeinrichtung ferner eine oder die genannte zweite flexible Rohreinrichtung (48) aufweist, die zwischen dem Flansch (46) und dem proximalen Anschlußstück (14) angeordnet und koaxial damit ausgefluchtet ist, um einen freien Durchgang durch die kollabierte Abschirmeinrichtung (16) zu schaffen und um eine Beanspruchung eines hindurchtretenden Katheters (22) zu verringern.

11. Einstellbare Katheterkontaminationsabschirmung nach einem der vorhergehenden Ansprüche,
wobei das distale Anschlußstück (12) die flexible Rohreinrichtung (76) aufweist, die koaxial damit ausgefluchtet ist, um eine Beanspruchung eines hindurchtretenden Katheters (22) zu verringern.

12. Einstellbare Katheterkontaminationsabschirmung nach einem der Ansprüche 1 bis 4 oder einem der Ansprüche 6 bis 11 in Abhängigkeit von einem der Ansprüche 1 bis 4,
wobei der eine Bereich zwischen dem distalen und dem proximalen Anschlußstück (12, 14) positioniert ist.

13. Einstellbare Katheterkontaminationsabschirmung nach einem der Ansprüche 1 bis 11,
wobei der eine Bereich an einem Ende der kollabierten Konfiguration angeordnet ist.

14. Verfahren zum Bereitstellen einer sterilen Umgebung außerhalb eines Körpers für einen Katheter (22), das die folgenden Schritte aufweist:
Koppeln eines distalen Anschlußstücks (12) für eine Kontaminationsabschirmung (16) mit einer Einführeinrichtung (32), wobei die Einführeinrichtung zum Einführen in ein Venenlumen (30) ausgebildet ist;
Führen des Katheters durch ein proximales Anschlußstück (14) und in einen sterilen Bereich (18), der von einer ausdehnbaren Abschirmung (16) umschlossen ist, die zwischen dem distalen und dem proximalen Anschlußstück angeordnet ist;
Ausdehnen der Abschirmung ausgehend von einem Ende einer kollabierten Konfiguration davon, um eine Sichtbeobachtung eines Katheterbereichs zu ermöglichen, der zwischen dem distalen und dem proximalen Anschlußstück verläuft.

## Revendications

1. Protection réglable contre la contamination pour cathéter comprenant un embout éloigné (12) comportant des moyens formant douille (96) pour accouplement à un dispositif d'introduction (32) et des moyens (76) définissant un alésage (92) dans l'embout éloigné, pour permettre le passage d'un cathéter (22) au travers de celui-ci ; un embout proche (14) comportant des moyens (48) définissant un alésage (90) dans celui-ci, pour permettre le passage d'un cathéter au travers de l'embout proche ; et des moyens formant protection repliables (16), disposés entre lesdits embouts éloigné et proche, pour définir un espace réglable (18) entre eux et pour protéger une partie du cathéter disposée entre les embouts éloigné et proche ; caractérisée par la présence de moyens formant support (46) pour supporter les moyens formant protection (16) à l'état replié et pour faire en sorte que les moyens formant protection à l'état repliés ne se déploient qu'à partir uniquement d'une partie de leur configuration à l'état replié, lorsque les embouts éloigné et proche sont écartés l'un de l'autre.

2. Protection réglable contre la contamination pour cathéter selon la revendication 1, dans laquelle lesdits moyens formant protection repliables (16) sont réalisés dans une matière permettant l'observation visuelle du cathéter (22) au travers de ladite partie déployée des moyens formant protection, lorsque le cathéter est disposé entre les embouts éloigné et proche (12, 14) ; et lesdits moyens formant support (46) sont actionnables pour produire la partie déployée, tout en conservant la partie restante des moyens formant protection à l'état replié, au fur et à mesure que les embouts éloigné et proche sont écartés l'un de l'autre.

3. Protection réglable contre la contamination pour cathéter selon la revendication 2, dans laquelle les moyens formant protection (16) permettent l'observation visuelle d'un cathéter (22) par la partie déployée de celle-ci, lorsque la partie déployée se trouve à l'état tendu ; et lesdits moyens formant support (46) sont raccordés à un desdits embouts éloigné et proche (12, 14) pour supporter les moyens formant protection à l'état replié sur eux-mêmes lorsque lesdits embouts éloigné et proche sont adjacents l'un par rapport à l'autre, pour permettre au cathéter d'être enfilé dans les alésages des embouts éloigné et proche pour faire en sorte que les moyens formant protection à l'état replié se déploient suivant ledit axe longitudinal lorsque les embouts éloigné et proche sont écartés l'un de l'autre et pour faire en sorte que la partie déployée de la protection soit dans un état de tension permettant l'observation visuelle du cathéter au travers de celle-ci.

4. Protection réglable contre la contamination pour cathéter selon l'une quelconque des revendications qui précèdent, dans laquelle les moyens (76) définissant un alésage (92) dans l'embout éloigné sont des premiers moyens formant tube souple (76) alignés de façon coaxiale avec ledit embout éloigné (12), empêchant ledit cathéter (22) d'être soumis à des sollicitations ; et les moyens (48) définissant l'alésage (90) dans l'embout porche (14) sont des seconds moyens formant tube souple (48) alignés de façon coaxiale avec ledit embout proche pour empêcher ledit cathéter d'être soumis à des sollicitations ; lesdits moyens formant protection repliables (16) étant disposés sur lesdits premiers et seconds moyens formant tube souple (76, 48).

5. Protection réglable contre la contamination pour cathéter selon la revendication 4, dans laquelle les seconds moyens formant tube souple (48) comportent lesdits moyens formant support (46), en utilisation, les moyens formant support (46) amenant les moyens formant protection à l'état replié (16) à se déployer selon l'axe longitudinal de ceux-ci à partir d'une extrémité de la configuration correspondant à l'état replié, lorsque les embouts éloigné et proche (12, 14) sont écartés l'un de l'autre.

6. Protection réglable contre la contamination pour cathéter selon l'une quelconque des revendications qui précèdent, dans laquelle lesdits moyens formant support (46) sont disposés près dudit embout proche (14).

7. Protection réglable contre la contamination pour cathéter selon l'une quelconque des revendications qui précèdent, dans laquelle lesdits moyens formant support (46) sont interconnectés avec ledit embout proche (14).

8. Protection réglable contre la contamination pour cathéter selon l'une quelconque des revendications qui précèdent, dans laquelle lesdits moyens formant support (46) sont disposés à l'intérieur desdits moyens formant protection (16).

9. Protection réglable contre la contamination pour cathéter selon l'une quelconque des revendications qui précèdent, dans laquelle lesdits moyens formant support comportent une bride (46) ayant un diamètre extérieur D suffisant pour empêcher le passage des moyens formant protection (16) au-dessus d'eux en l'absence de tout mouvement des embouts éloigné et proche (12, 14) les faisant s'écarter l'un de l'autre.

10. Protection réglable contre la contamination pour cathéter selon la revendication 9, dans laquelle lesdits moyens formant support comportent en outre des ou dits second moyens formant tube souple (48) disposés entre ladite bride (46) et l'embout proche (14) et alignés de façon axiale avec ceux-ci, pour assurer un passage dégagé au travers des moyens formant protection à l'état replié (16) et pour réduire les sollicitations imposées à un cathéter (22) traversant ceux-ci.

11. Protection réglable contre la contamination pour cathéter selon l'une quelconque des revendications qui précèdent, dans laquelle ledit embout éloigné (12) comporte des moyens formant tube souple (76) alignés de façon coaxiale avec celui-ci, pour réduire les sollicitations imposées à un cathéter (22) traversant celui-ci.

12. Protection réglable contre la contamination pour cathéter selon l'une quelconque des revendications 1 à 4 ou l'une quelconque des revendications 6 à 11 lorsqu'elles dépendent de l'une quelconque des revendications 1 à 4, dans laquelle ladite partie est positionnée entre les embouts éloigné et proche (12, 14).

13. Protection réglable contre la contamination pour cathéter selon l'une quelconque des revendications 1 à 11, dans laquelle ladite partie est disposée à l'extrémité de la configuration correspondant à l'état replié.

14. Procédure pour assurer un environnement stérile à l'extérieur d'un corps pour un cathéter (22) comprenant les étapes consistant à :
accoupler un embout éloigné (12) pour une protection contre la contamination (16) à un dispositif d'introduction (32), le dispositif d'introduction étant conçu pour introduction dans un passage veineux (30) ;
faire passer le cathéter au travers d'un embout proche (14) et dans une enceinte stérile (18) définie par une protection expansible (16) disposée entre lesdits embouts éloigné et proche ;
déployer la protection à partir d'une extrémité correspondant à la configuration repliée de celle-ci pour permettre l'observation visuelle de la partie du cathéter passant entre les embouts éloigné et proche.
